(19) Europäisches Patentamt
European Patent Office
Office européen des brevets

(11) Numéro de publication: **0 223 620 B1**

# (12) FASCICULE DE BREVET EUROPEEN

(45) Date de publication du fascicule du brevet: **12.04.89**

(51) Int. Cl.[4]: **C 07 C 53/18**, C 07 C 51/363, C 07 C 69/63, C 07 C 67/307

(21) Numéro de dépôt: **86401891.6**

(22) Date de dépôt: **28.08.86**

(54) **Procédé de préparation de l'acide bromo-fluoro-acétique.**

(30) Priorité: **13.09.85 FR 8513611**

(43) Date de publication de la demande: **27.05.87 Bulletin 87/22**

(45) Mention de la délivrance du brevet: **12.04.89 Bulletin 89/15**

(84) Etats contractants désignés: **AT BE CH DE FR GB IT LI LU NL SE**

(56) Documents cités:
**FR-A- 1 381 853**

**CHEMICAL ABSTRACTS, vol. 90, no. 3, 15 janvier 1979, page 586, résumé no. 22332v, Columbus, Ohio, US**

(73) Titulaire: **ATOCHEM, 4 & 8, Cours Michelet La Défense 10, F-92800 Puteaux (FR)**

(72) Inventeur: **Drivon, Gilles, 3, rue Joany Courbiere, F-69850 Saint-Martin-en-Haut (FR)**
Inventeur: **Gurtner, Bernard, 5, Boulevard Agutte Sembat, F-38000 Grenoble (FR)**

(74) Mandataire: **Leboulenger, Jean et al, ATOCHEM Département Propriété Industrielle, F-92091 Paris la Défense 10 Cédex 42 (FR)**

**EP 0 223 620 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne la synthèse de l'acide bromo-fluoro-acétique CHFBr-COOH utile comme matière première pour la fabrication de médicaments et de produits phytosanitaires.

La synthèse de l'acide CHFBr-COOH à partir du trifluorochloroéthylène a été réalisée par HASZELDINE (J. Chem. Soc. 1952, 4259) en 7 étapes successives et avec un rendement global de seulement 18% selon le schéma réactionnel suivant:

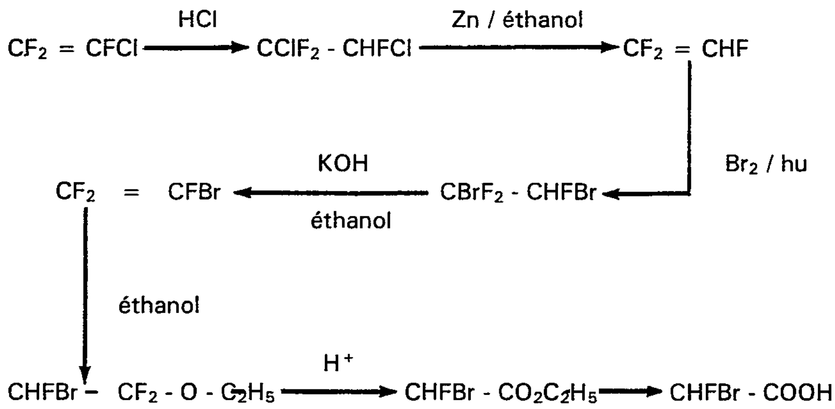


Le brevet FR 1 381 853 (DOW) a pour objet un «Procédé de préparation de bromures aliphatiques et d'acides aliphatiques bromés» consistant à faire réagir un chlorure aliphatique ou un acide aliphatique chloré avec le gaz bromhydrique en présence d'acide bromhydrique aqueux à une température comprise entre le voisinage de la température ambiante et environ 140°C. Toutefois ce brevet ne mentionne pas la possibilité pour le produit de départ de contenir un atome de fluor.

Il a maintenant été trouvé qu'on peut obtenir plus simplement cet acide à partir de la même matière première ($CF_2 = CFCl$) en seulement trois étapes et avec un rendement global amélioré, en soumettant un ester de l'acide chlorofluoroacétique ($CHClF - CO_2R$) en solution dans l'acide bromhydrique concentré, à l'action du bromure d'hydrogène gazeux, ledit ester étant préparé de façon connue par addition d'un alcool (ROH) sur le trifluorochloroéthylène et hydrolyse en milieu acide de l'éther $CHFCl-CF_2-OR$ (voir «aliphatic Fluorine Compounds» de A.M. LOVELACE et al, Reinhold Publishing Corp., 1958, pages 157 et 234).

La troisième étape définie ci-dessus (action de HBr gazeux sur $CHFCl-CO_2R$ en milieu bromhydrique concentré) peut s'appliquer aux composés chlorofluorés de formule:

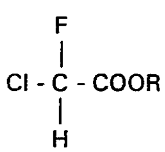
(I)

dans laquelle R représente un atome d'hydrogène, un radical alkyle (de préférence, méthyle ou éthyle), ou un radical aryle.

L'invention a donc pour objet un procédé de préparation de l'acide bromo-fluoro-acétique de formule:

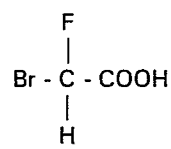
(II)

caractérisé en ce que l'on soumet à l'action du bromure d'hydrogène gazeux un composé de formule (I) en solution dans l'acide bromhydrique concentré.

On dissout le composé (I) dans l'acide bromhydrique concentré, puis la réaction est effectuée sous agitation en chauffant le mélange réactionnel et en introduisant le bromure d'hydrogène gazeux. L'acide chlorhydrique HCl étant insoluble dans ce milieu se dégage sous forme gazeuse, en même temps qu'un peu de bromure d'hydrogène.

Il est avantageux d'utiliser comme radical R un méthyle ou éthyle, et de chauffer à reflux pour éliminer le bromure d'alkyle correspondant et ainsi accélérer la réaction.

L'acide bromhydrique concentré initial peut avoir une concentration de 45 à 60% en poids; de préférence on utilise une solution commerciale à 48%.

On opère de préférence sous la pression atmosphérique, en maintenant la température du mélange réactionnel entre 50 et 140°C, avantageusement entre 80 et 125°C.

Le bromure d'hydrogène gazeux est de préférence introduit à un débit permettant sa consommation complète. L'avancement de la réaction peut être suivi par la mesure du HCl dégagé. La réaction terminée, on peut isoler l'acide bromo-fluoro-acétique formé, par distillation sous vide du mélange réactionnel.

Bien qu'on préfère dissoudre le composé de formule (I) directement dans l'acide bromhydrique concentré, on ne sortirait pas du cadre de la présente invention en mélangeant d'abord le composé (I) avec de l'eau et en injectant dans ce mélange du bromure

d'hydrogène gazeux de façon à former in situ le milieu acide bromhydrique concentré.

Les exemples suivants illustrent l'invention sans la limiter.

*Exemple 1*

On opère dans un réacteur en verre de 0,5 litre muni d'un agitateur rotatif, d'un thermomètre, d'un injecteur de gaz et d'une colonne à garnissage terminée par un réfrigérant refroidi par une saumure dont la température est régulée entre −10 et −15°C, la sortie dudit réfrigérant étant équipée d'un séparateur gaz liquide dont la phase gazeuse est reliée à un piège à eau où l'on dose HCl. On introduit dans ce réacteur 211 g de chlorofluoroacétate d'éthyle $CFClH\text{-}COOC_2H_5$ (soit 1,5 mole) et 148 g d'acide bromhydrique à 48% (soit 0,1 litre). On chauffe à 90°C et on commence à injecter le HBr gazeux à un débit de 0,3 mole/heure environ. La puissance de chauffage est ajustée pour avoir un reflux en tête de la colonne de distillation. Après 7 heures de réaction (durée comptée à partir du moment où la température du mélange réactionnel a atteint 90°C), on a recueilli en sortie du réfrigérant 150 g de bromure d'éthyle et la température est alors de 105°C. Après 13 h 30, la température ayant atteint 120°C et l'acide chlorhydrique dosé dans le piège correspondant à la quantité théorique (soit 1,5 mole), on coupe l'injection de bromure d'hydrogène gazeux. On étête sous un vide de 50 torrs absolus l'acide bromhydrique (plus précisément l'azéotrope HBr - $H_2O$) et on obtient ainsi un acide CHFBr-COOH brut qu'on identifie et dose par RMN. On récupère par distillation du coeur, sous un vide de 22 torrs absolus, 168 g d'acide pur.

*Exemple 2*

Dans le même appareillage que dans l'exemple 1, mais dont on a enlevé la recette liquide en sortie du réfrigérant, on introduit 190 g de chlorofluoroacétate de méthyle $CHClFCOOCH_3$ (soit 1,5 mole) et 148 g d'acide bromhydrique à 48%. On chauffe à 80°C à reflux et on injecte 0,3 mole/h de bromure d'hydrogène gazeux comme dans l'exemple 1. En sortie du réfrigérant la phase gazeuse contient: HBr, HCl et $CH_3Br$.

Après 10 h la température du mélange réactionnel est de 110°C. Après 15 h on a obtenu la quantité théorique d'HCl soit 1,5 moles. On coupe alors l'injection de bromure d'hydrogène gazeux et l'acide bromhydrique est étêté sous vide.

On obtient un acide brut qu'on identifie et dose par RMN. Par distillation du coeur sous un vide de 62 torrs absolus à 116°C on récupère 192 g d'acide pur CHFBr-COOH.

*Exemple 3*

Dans le même appareillage que dans l'exemple 1, on introduit 169 g de CHFCl-COOH (soit 1,5 mole) et 195 g d'acide bromhydrique à 48%. On chauffe le mélange à 80°C et on injecte pendant 5 heures, du bromure d'hydrogène gazeux à un débit de 0,5 mole/h. La température dans le réacteur en fin d'essai est de 120°C. On récupère ainsi 1,2 mole d'HCl dans le piège à eau.

L'acide bromhydrique est étêté sous vide. Après analyse et distillation, comme dans les exemples précédents, on récupère 1 mole d'acide CHFBr-COOH.

## Revendications

1. Procédé de préparation de l'acide bromofluoroacétique de formule:

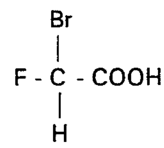

caractérisé en ce que l'on soumet un composé de formule:

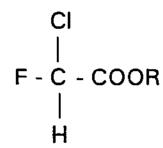

dans laquelle R représente un atome d'hydrogène ou un radical alkyle ou aryle à l'action du bromure d'hydrogène gazeux en milieu aqueux bromhydrique concentré.

2. Procédé selon la revendication 1, caractérisé en ce que R est un atome d'hydrogène ou un radical méthyle ou éthyle.

3. Procédé selon la revendication 1 ou 2 dans lequel R est un radical alkyle et le bromure d'alkyle est éliminé au fur et à mesure de sa formation.

4. Procédé selon l'une des revendications 1 à 3 dans lequel le milieu bromhydrique concentré est de l'acide bromhydrique en solution aqueuse entre 45 et 60% en poids et, de préférence, à 48% en poids.

5. Procédé selon l'une des revendications 1 à 4 dans lequel la température du mélange réactionnel est comprise entre 50 et 140°C et, de préférence, entre 80 et 125°C.

6. Procédé selon l'une des revendications 1 à 5 dans lequel on opère à la pression atmosphérique.

## Patentansprüche

1. Verfahren zur Herstellung von Bromfluoressigsäure der Formel:

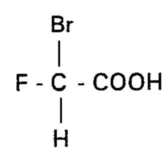

dadurch gekennzeichnet, daß man eine Verbindung der Formel:

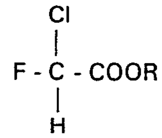

in der R ein Wasserstoffatom oder einen Alkylrest oder einen Arylrest darstellt, der Einwirkung von gasförmigem Bromwasserstoff in wäßrigem konzentriertem Bromwasserstoff-Milieu aussetzt.

2. Verfahren nach Anspruch 1, dadurch gekennzeichnet, daß R ein Wasserstoffatom oder ein Methylrest oder Ethylrest ist.

3. Verfahren nach Anspruch 1 oder 2, bei dem R ein Alkylrest ist und das Alkylbromid nach und nach bei seiner Bildung entfernt wird.

4. Verfahren nach einem der Ansprüche 1 bis 3, bei dem das konzentrierte Bromwasserstoff-Milieu Bromwasserstoffsäure in wäßriger Lösung mit zwischen 45 und 60 Gew.-% und vorzugsweise mit 48 Gew.-% ist.

5. Verfahren nach einem der Ansprüche 1 bis 4, bei dem die Temperatur des Reaktionsgemisches zwischen 50 und 140°C und vorzugsweise zwischen 80 und 125°C liegt.

6. Verfahren nach einem der Ansprüche 1 bis 5, bei dem man bei atmosphärischem Druck arbeitet.

**Claims**

1. Process for the preparation of bromofluoroacetic acid of formula:

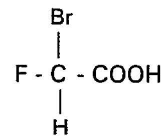

characterized in that a compound of formula:

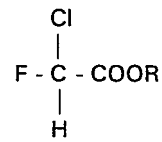

in which R represents a hydrogen atom or an alkyl or aryl radical, is reacted with gaseous hydrogen bromide in a concentrated hydrobromic acid medium.

2. Process according to Claim 1, characterized in that R is a hydrogen atom or a methyl or ethyl radical.

3. Process according to Claim 1 or 2 in which R is an alkyl radical and the alkyl bromide is removed as and when it is formed.

4. Process according to one of Claims 1 to 3 in which the concentrated hydrobromic medium is a 45 to 60% by weight, and preferably 48% by weight, aqueous solution of hydrobromic acid.

5. Process according to one of Claims 1 to 4 in which the temperature of the reaction mixture is between 50 and 140°C, and preferably between 80 and 125°C.

6. Process according to one of Claims 1 to 5 in which the reaction is carried out at atmospheric pressure.